# EUROPÄISCHE PATENTSCHRIFT

(11) **EP 0 891 160 B1**
(45) Veröffentlichungstag und Bekanntmachung des Hinweises auf die Patenterteilung: **24.10.2001**
(21) Anmeldenummer: 97916374.8
(22) Anmeldetag: 21.03.1997
(51) Int. Cl.: A61B 17/68

(54) **IMPLANTAT ZUR FIXIERUNG EINER OSTEOTOMIE**
IMPLANT FOR FIXING BONE FRAGMENTS AFTER AN OSTEOTOMY
IMPLANT POUR FIXER DES FRAGMENTS D'OS APRES UNE OSTEOTOMIE

(30) Priorität: 26.03.1996 AT 16796 U
(43) Veröffentlichungstag der Anmeldung: 20.01.1999
(73) Patentinhaber: Waldemar Link (GmbH & Co.), 22339 Hamburg (DE)
(72) Erfinder: STOFFELLA, Rudolf, A-1010 Wien (AT)
(74) Vertreter: Glawe, Delfs, Moll & Partner
(86) Internationale Anmeldenummer: EP9701452
(87) Internationale Veröffentlichungsnummer: WO9735528

(56) Entgegenhaltungen:
- EP-A- 0 261 038
- EP-A- 0 401 650
- EP-A- 0 646 353
- FR-A- 2 722 545

## Beschreibung

Die Erfindung betrifft ein Implantat zur Fixierung einer Osteotomie, insbesondere zur Behandlung einer Achsenfehlstellung eines Mittfußknochens, z. B. Hallux valgus, bestehend aus einer Spange mit zwei Schenkeln, die an ihren einen Enden miteinander verbunden sind.

Osteotomien zur Behandlung des Hallux valgus sind bereits seit Jahrzehnten bekannt und haben das Ziel, die Achse der Metatarsale l wieder anatomisch zu rekonstruieren. Hierbei ist es erforderlich, nach dem Reponieren der Osteotomie die beiden Knochenfragmente in ihrer reponierten Stellung zu fixieren, um eine interfragmentäre Beweglichkeit zu verhindern und die primäre Knochenheilung ohne Unruhekallus zu ermöglichen. Für diese Fixierung gibt es verschiedene Techniken.

So ist es bereits bekannt, die beiden Knochenfragmente durch eine Schraube miteinander zu verbinden, die in ein vorgebohrtes Gleitloch des proximalen Fragmentes eingeführt und in ein Gewindeloch des distalen Fragmentes eingeschraubt wird. Auch die Verwendung von selbstschneidenden Schrauben oder von Hohlschrauben, welche eine temporäre Fixation der Osteotomie durch zum System gehörende Kirschnerdrähte ermöglicht, ist bereits bekannt. Die Verwendung dieser Schrauben bewirkt eine starke interfragmentäre Kompression, welche sich aber nicht als vorteilhaft erwiesen hat, da ein Druck von mehr als 206,8 KPa (30 psi) zu einer Kortikalisnekrose führt.

Es wurde bereits vorgeschlagen, eine Osteotomie durch einen Draht zu fixieren, der durch vorgebohrte Löcher in der Osteotomie geschoben wird, worauf die Drahtenden verzwirbelt werden und der verzwirbelte Bereich in einem gesonderten Loch im Knochen versenkt wird. Diese Art der Fixierung ist umständlich und aufwendig.

Die Fixierung der Osteotomie mit einem aus der Haut hervorragenden Bohrdraht bewirkt eine temporäre Unbeweglichkeit des Großzehengrundgelenks, was einen Nachteil darstellt.

Zur Fixierung der Osteotomie sind auch Knochenklammem bekannt, deren Anwendung jedoch die Gefahr der Knochenabsplitterungen mit sich bringt.

Ferner werden zur Fixierung der Osteotomie Platten verwendet, die über mehrere Schrauben an den Kortikalen befestigt werden müssen, so daß mehr Knochenmasse verlorengeht und ein erheblicher operativer Aufwand hierfür erforderlich ist.

Es ist eine Osteosynthesespange bekannt (FR-A-2722545), die aus zwei parallelen Schenkeln besteht, die U-förmig über einen einzigen Bogen ohne Umkehrpunkt miteinander verbunden sind. Mit dem Bogen der Spange erfaßt man ein Knochenfragment und sticht die freien Schenkelenden in das andere Fragment, wo sie dank ihrer Wellung gehalten werden sollen.

Ausgehend von dem letztgenannten Stand der Technik will die Erfindung ein Implantat zur Fixierung einer Osteotomie schaffen, das insbesondere zur Behandlung einer Achsenfehlstellung eines Mittelfußknochens geeignet ist und eine ausreichend stabile Fixierung der Osteotomie sicherstellt, leicht und ohne Irritationen einsetzbar und im Knochen verankerbar ist und auch patientenfreundlich wieder entfernt werden kann.

Die erfindungsgemäße Lösung besteht in den Merkmalen des Anspruchs 1. Demgemäß ist vorgesehen, daß die beiden Schenkel im Bereich ihrer miteinander verbundenen Enden eine Öse zum Hindurchführen einer in einem der beiden Knochenfragmente verankerbaren Schraube begrenzen. Die anderen, freien Enden der Schenkel sind im Markraum des anderen Knochenfragmentes ausspreizbar. Diese Verspreizung der Schenkel im Markraum des Knochens wird dadurch erzielt, daß der Abstand zwischen den beiden aus einem biegeelastischen Material, beispielsweise aus Stahldraht, bestehenden Schenkeln zumindest im unbelasteten, entspannten Zustand von der Verbindungsstelle ausgehend in Richtung sich zu ihren freien Enden hin vergrößert. Beim Einführen der Schenkel in den Markraum werden diese zusammengedrückt und verspreizen sich nach Aufhören des auf die Schenkel ausgeübten Drucks infolge ihrer biegeelastischen Eigenschaften an der Wand des Markraumes.

Nach erfolgter Osteotomie wird die Spange mit den freien Enden ihrer beiden Schenkel in den Markraum nach proximal eingesetzt und verspreizt sich dort im Markraum des einen Knochenfragmentes, worauf das mit der Öffnung versehene Ende der Spange durch die Schraube am anderen Knochenfragment verankert wird. Durch diese intramedulläre Verankerung der Spange mittels der ausgespreizten Schenkelenden einerseits und die Verschraubung der Spange andererseits bekommt die Osteotomie eine ausreichend stabile Fixierung und ermöglicht eine sofortige Belastbarkeit und Frühmobilisation mit einer Hallux-Motorschiene. Eine aufwendige Wundpflege und Zügelverbände, die die Beweglichkeit einschränken, sind nicht mehr erforderlich.

Die Spange und die Schraube können ab der vierten postoperativen Woche mit einer Stichinzision patientenfreundlich entfernt werden.

Zweckmäßigerweise ist die Schraubenöse mit einer im wesentlichen kreisbogenförmigen Begrenzung ausgebildet, die so bemessen ist, daß die Schraube durch diese Öffnung hindurchgeführt werden kann, jedoch eine unerwünschte laterale Bewegung der Schraube innerhalb der Öffnung verhindert wird, und durch den Schraubenkopf ein Anpressen an das Knochenfragment erfolgt.

Weiter ist es von Vorteil, wenn die die Öffnung begrenzenden, miteinander verbundenen Enden der beiden Schenkel aus der vom übrigen Teil der Schenkel aufgespannten Schenkelebene herausgebogen sind und in einer zu dieser Schenkelebene etwa parallelen Ebene liegen. Der Abstand zwischen diesen beiden Ebenen berücksichtigt die laterale Versetzung der Knochenfragmente.

Die intramedulläre Verankerung läßt sich dadurch verbessern, daß erfindungsgemäß die beiden Schenkel eine, vorzugsweise in der Schenkelebene verlaufende, Wellung aufweisen.

Es kann aber auch die Spange aus einem Memory-Metall, vorzugsweise aus einer Nickel-Titan-Legierung bestehen. Derartige Memory-Metalle sind bekannt und werden auch für Implantate verwendet Diese Memory-Metalle haben die Eigenschaft, bei einer mechanischen Verformung unter bestimmten Temperaturen ihre durch diese mechanische Verformung bewirkte Gestalt beizubehalten, jedöch bei einem Ansteigen der Temperatur in ihre ursprüngliche Gestalt zurückzukehren. Besteht also die Spange aus einem Memory-Metall, so können nach Abkühlen der Spange beispielsweise durch Aufsprühen eines Kühlgases die beiden Schenkelenden einander angenähert werden und behalten zunächst diese Form bei. Nach dem Einführen der Schenkel in den Markraum erhöht sich die Temperatur der Spange auf Körpertemperatur, worauf die aus dem Memory-Metall bestehende Spange wieder ihre ursprüngliche Form mit ausgespreizten Schenkelenden einnimmt und sich dadurch an der Wand des Markraumes verspreizt.

In der Zeichnung ist die Erfindung anhand eines Ausführungsbeispieles schematisch veranschaulicht. Fig. 1 zeigt die Verwendung des erfindungsgemäßen Implantates bei der Rekonstruktion eines Hallux valgus. Fig. 2 stellt das erfindungsgemäße Implantat in Draufsicht und Fig. 3 in Seitenansicht dar.

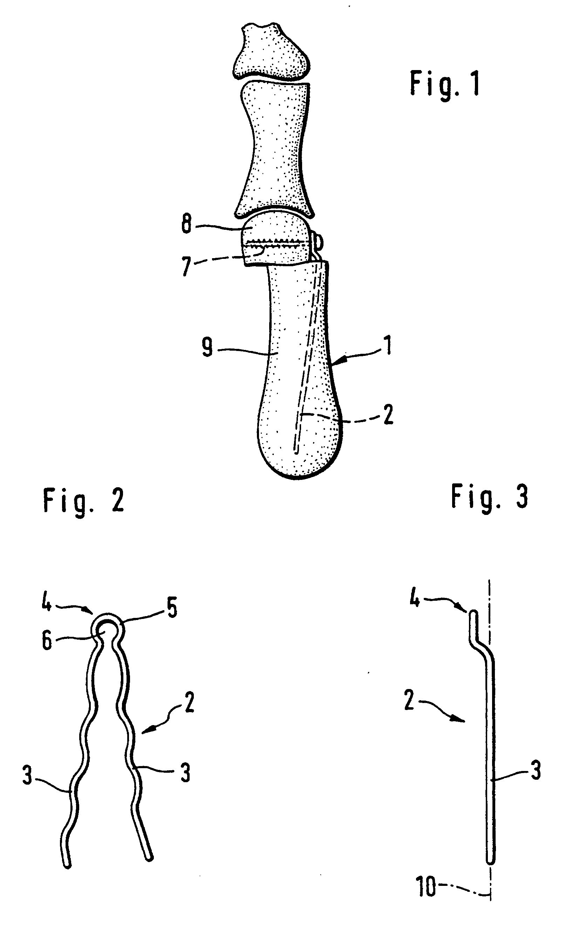

In Fig. 1 ist ein Mittelfußknochen 1 nach erfolgter Osteotomie und Reposition dargestellt. Zur Fixierung wird ein Implantat verwendet, das erfindungsgemäß aus einer in den Fig. 2 und 3 dargestellten Spange 2 besteht. Diese Spange 2 weist zwei Schenkel 3 auf, deren eine Enden bei 4 miteinander verbunden sind und dort eine von einer Öse 5 gebildete Öffnung 6 begrenzen, durch die eine Kleinfragmentschraube 7 (siehe Fig. 1) hindurchführbar ist, die im Knochenfragment 8 in üblicher Weise verankert wird.

Die beiden Schenkel 3 der Spange 2 werden in den Markraum des Knochenfragmentes 9 eingeführt und nehmen dort ihre in Fig. 2 dargestellte ausgespreizte Lage ein, wodurch die Spange 2 in diesem Markraum verankert wird. Die Verankerung wird durch eine vorzugsweise in der Schenkelebene verlaufene Wellung unterstützt.

Wie aus den Fig. 1 und 3 hervorgeht, sind die die Öffnung 6 begrenzenden, miteinander bei 4 verbundenen Enden der beiden Schenkel aus der vom übrigen Teil der Schenkel 3 aufgespannten Schenkelebene 10 herausgebogen und liegen in einer zu dieser Schenkelebene 10 parallelen Ebene. Dadurch wird die laterale Verschiebung der beiden Knochenfragmente 8,9 berücksichtigt. Der Abstand zwischen diesen Ebenen ist also dieser lateralen Verschiebung angepaßt.

Die Spange 2 kann aus einem biegeelastischen Stahldraht bestehen, wobei sich der Abstand der beiden Schenkel 3 im unbelasteten Zustand von der Verbindungsstelle 4 ausgehend in Richtung zu den freien Enden der Schenkel vergrößert. Beim Einführen der Schenkel 3 in den Markraum werden diese zusammengedrückt und verspreizen sich nach Beendigung des manuell auf die Schenkel ausgeübten Druckes im Markraum. Es kann aber auch die Spange 2 aus einem Memory-Metall, vorzugsweise aus einer Nickel-Titan-Legierung bestehen, also aus einem Metall. das infolge seiner kristallinen Struktur der Legierung in der austenitischen Phase bei tiefen Temperaturen die mechanisch erfolgte Deformation beibehält, bei Erwärmung jedoch infolge des Erinnerungsvermögens in seine ursprüngliche Form zurückkehrt. Im vorliegenden Fall ist die ursprüngliche Form in Fig. 2 dargestellt Bei Abkühlung, beispielsweise mittels eines aufgesprühten Kühlgases können jedoch die Schenkel mechanisch in eine etwa parallel verlaufende Gestalt gebracht werden und behalten diese Gestalt während des Einführens in den Markraum bei, wodurch dieses Einführen erleichtert wird. Erwärmt sich die Spange durch die Körpertemperatur, so kehren die Schenkel in ihre ursprüngliche, in Fig. 2 dargestellte Form zurück und verspreizen sich im Markraum. Diese Vorgangsweise erleichtert somit das Einführen der Schenkel 3 der Spange 2 in den Markraum.

## Patentansprüche

1. Implantat zur Fixierung einer Osteotomie, insbesondere zur Behandlung einer Achsenfehlstellung eines Mittelfußknochens, z.B. Hallux valgus, bestehend aus einer im wesentlichen V-förmigen Spange (2) mit zwei in den Markraum eines von zwei Knochenfragmenten einführbaren Schenkeln (3) aus biegeelastischem Material, die an ihren Enden miteinander verbunden sind, wobei die beiden Schenkel (3) im Bereich ihrer miteinander verbundenen Enden eine Schraubenöse (5) zum Hindurchführen einer in dem anderen der beiden Knochenfragmente verankerbaren Schraube (7) bilden und ihr Abstand sich im entspannten Zustand in Richtung zu ihren freien Enden hin derart vergrößert, daß sie nach Implantation im zusammengedrückten Zustand im Markraum des einen Knochenfragments sich darin zu verspreizen in der Lage sind.

2. Implantat nach Anspruch 1, **dadurch gekennzeichnet, daß** die Schraubenöse (5) mit einer im wesentlichen kreisbogenförmigen Begrenzung ausgebildet ist.

3. Implantat nach Anspruch 1 oder 2, **dadurch gekennzeichnet, daß** die die Schraubenöse (5) bildenden, miteinander verbundenen Enden der beiden Schenkel (3) aus der vom übrigen Teil der Schenkel (3) aufgespannten Schenkelebene (10) herausgebogen sind und in einer zu dieser Schenkelebene (1) etwa parallelen Ebene liegen.

4. Implantat nach Anspruch 1, 2 oder 3, **dadurch gekennzeichnet, daß** die beiden Schenkel (3) eine vorzugsweise in der Schenkelebene verlaufende Wellung aufweisen.

5. Implantat nach einem der Ansprüche 1 bis 4, **dadurch gekennzeichnet, daß** die Spange (2) aus einem Memory-Metall, vorzugsweise aus einer Nickel-Titan-Legierung, besteht.

## Claims

1. An implant for fixing an osteotomy, in particular for treating an axial malposition of a metatarsal bone, for example hallux valgus, comprising a substantially V-shaped clip (2) having two arms (3) of flexible material which can be inserted into the medullary cavity of one of two bone fragments and which are joined together at their ends, wherein in the vicinity of their joined together ends the two arms (3) form a screw eyelet (5) for the passage of a screw (7) which can be anchored in the other of the two bone fragments, and their distance apart in the relaxed position increases towards their free ends in such a way that, after implantation in the compressed condition in the medullary cavity of one bone fragment, they are able to expand therein.

2. An implant according to Claim 1, **characterised in that** the screw eyelet (5) is formed with a substantially circular-arc shaped boundary.

3. An implant according to Claim 1 or 2, **characterised in that** the joined together ends of the two arms (3) forming the screw eyelet (5) are bent out of the plane (10) of the arms extending from the remaining portion of the arms (3) and lie in a plane approximately parallel to this arm plane (10).

4. An implant according to Claim 1, 2 or 3, **characterised in that** the two arms (3) have a corrugation preferably extending in the plane of the arms.

5. An implant according to any one of Claims 1 to 4, **characterised in that** the clip (2) is formed from a shape memory metal, preferably from a nickel-titanium alloy.

## Revendications

1. Implant pour la fixation d'une ostéotomie, notamment pour traiter une mauvaise position de l'axe d'un os du métatarse, par exemple hallux valgus, constitué d'une agrafe (2) sensiblement en forme de V avec deux branches (3) à introduire dans le canal médullaire de l'un de deux fragments d'os, constituées d'une matière élastique et reliées entre elles à leurs extrémités, les deux branches (3) formant, dans la zone de leurs extrémités reliées, un oeillet à vis (5) pour le passage d'une vis (7) qui peut être ancrée dans l'autre des deux fragments d'os, et leur distance à l'état détendu augmentant en direction de leurs extrémités libres, de manière qu'après implantation à l'état comprimé dans le canal médullaire d'un fragment d'os, elles soient en mesure de s'écarter celui-ci.

2. Implant selon la revendication 1, **caractérisé en ce que** l'oeillet à vis (5) présente une délimitation sensiblement en forme d'arc de cercle.

3. Implant selon la revendication 1 ou 2, **caractérisé en ce que** les extrémités des deux branches (3), qui sont reliées entre elles et qui forment l'oeillet à vis (5), sont recourbées à l'extérieur du plan (10) des branches, défini par la partie restante des branches (3), et se situent dans un plan approximativement parallèle à ce plan (10) des branches.

4. Implant selon la revendication 1, 2 ou 3, **caractérisé en ce que** les deux branches (3) présentent une ondulation qui s'étend de préférence dans le plan des branches.

5. Implant selon l'une des revendications 1 à 4, **caractérisé en ce que** l'agrafe (2) est constituée d'un métal à mémoire, de préférence d'un alliage au nickel et titane.
